**Europäisches Patentamt**

**European Patent Office**

**Office européen des brevets**

(19)

(11) Veröffentlichungsnummer: **0 084 546**
**B1**

(12)

# EUROPÄISCHE PATENTSCHRIFT

(45) Veröffentlichungstag der Patentschrift:
25.09.85

(51) Int. Cl.⁴: **A 61 K 7/06**

(21) Anmeldenummer: **82902249.0**

(22) Anmeldetag: **14.07.82**

(86) Internationale Anmeldenummer:
**PCT/DE 82/00148**

(87) Internationale Veröffentlichungsnummer:
**WO 83/00433 (17.02.83** Gazette 83/5)

(54) **HAARPFLEGEMITTEL.**

(30) Priorität: **31.07.81 DE 3130894**

(43) Veröffentlichungstag der Anmeldung:
**03.08.83 Patentblatt 83/31**

(45) Bekanntmachung des Hinweises auf die Patenterteilung:
**25.09.85 Patentblatt 85/39**

(84) Benannte Vertragsstaaten:
**AT BE CH FR GB LI LU NL SE**

(56) Entgegenhaltungen:
**FR - A - 2 207 652**
**GB - A - 132 336**

**Chemical Abstracts, vol. 89, no. 8, 21 August 1978,**
**(Columbus, Ohio, US) see page 332, column 2, abstract**
**65139j, JP, A, 7844639, 21 April 1978, OGAWA**
**Ullmanns Encyclopädie der technischen Chemie Band**
**11 Seite 502**
**Hackh's Chemical Dictionary Seite 473**
**Römpps Chemie Lexikon, Seiten 2418-2419**

(73) Patentinhaber: **KRIWET, Manfred, Ansorgestrasse 8,**
**D-2000 Hamburg 52 (DE)**

(72) Erfinder: **KRIWET, Manfred, Ansorgestrasse 8,**
**D-2000 Hamburg 52 (DE)**

(74) Vertreter: **UEXKÜLL & STOLBERG Patentanwälte,**
**Beselerstrasse 4, D-2000 Hamburg 52 (DE)**

ACTORUM AG

## Beschreibung

Gegenstand der Erfindung sind Haarpflegemittel, wie Haarwasser, Haarwuchsmittel und Haarwaschmittel.

Haarpflegemittel, wie Haarwasser, Haarwuchsmittel und Haarwaschmittel zur Pflege der Haare und des Haarbodens (Kopfhaut) sind seit langem bekannt und existieren in einer unübersehbaren Vielzahl variierender Zusammensetzung (vgl. z. B. Taschenbuch der modernen Parfümerie und Kosmetik, Hugo Janistyn, 3. Aufl., 1966). Trotz vieler Versuche war die haarwuchsfördernde Wirkung dieser Mittel bisher nicht zufriedenstellend. Es wurde nun überraschend gefunden, dass derartigen Mitteln eine gute haarwuchsfördernde Wirkung verliehen werden kann, wenn ihnen bestimmte Extrakte zugesetzt werden.

Aus der GB-A Nr. 132336 ist ferner eine kosmetische Zusammensetzung bekannt, die überwiegend aus Olivenkernöl und im übrigen aus Pfirsichkernöl, Mandelöl und Wegerichöl sowie gegebenenfals Parfüm wie Fliederöl besteht. Diese ölige Zusammensetzung wird für die Massage und Behandlung des Gesichtes empfohlen. Als Haarpflegemittel kommt dieses Präparat aufgrund seiner Zusammensetzung ganz offensichtlich nicht in Frage.

Gegenstand der Erfindung sind dementsprechend Haarpflegemittel wie Haarwasser, Haarwuchsmittel und Haarwaschmittel der im Patentanspruch gekennzeichneten Art.

Die Herstellung der erfindungsgemäss zu verwendenden Extrakte erfolgt durch übliche Extraktion von Olivenkernen, Granatapfelsamen *(Punica granatum), Glossostemon bruguieri* oder Knollen der *Pueraria mirifica* (einer Leguminosenart). Dabei werden z. B. entfettete oder nicht entfettete Olivenkerne mit geeigneten organischen Lösungsmitteln wie Aceton, Methanol, n-Propanol, Isopropanol, Eisessig oder Gemischen derselben ausgezogen und daraus Fluid-Extrakte, Spissum-Extrakte oder Trockenextrakte hergestellt. Diese werden dann dem Haarpflegemittel zugesetzt. Üblicherweise werden die beispielsweise eingesetzten Olivenkerne vor der Extraktion gemahlen.

Zur Herstellung von Haarwasser werden die genannten pflanzlichen Bestandteile wie z. B. gemahlene Olivenkerne vorzugweise mit Isopropanol oder Ethanol extrahiert, so dass ggf. nach Entfernung überschüssigen Extraktionsmittels Fluid-Extrakte erhalten werden, die unmittelbar als Bestandteil von Haarwasser eingesetzt werden können. Gewöhnlich enthält derartiges Haarwasser weitere haarboden oder haarpflegende Substanzen, die dem Fachmann geläufig sind.

Bei der Herstellung von erfindungsgemässen Haarwasser werden meist 10 bis 25 Vol.-% und insbesondere 20 Vol.-% Fluid-Extrakt verwendet. Der Feststoffgehalt des Fluid-Extrakts beträgt im allgemeinen etwa 6%. Dementsprechend werden bei Verwendung von Spissum-Extrakt oder Trokkenextrakt geringere Mengen wie z. B. 4 bis 6% Trockenextrakt zur Herstellung von Haarwasser eingesetzt. In diesem Zusammenhang sei erwähnt, dass unter einem Fluid-Extrakt gewöhnlich ein Extrakt verstanden wird, bei dem das Verhältnis von zu extrahierender Substanz zu Extraktionsmittel 1:1 beträgt.

Die erfindungsgemässen Haarpflegemittel haben sich überraschenderweise als äusserst wirksam zur Förderung des Haarwuchses erwiesen. Dies soll anhand des folgenden Beispiels demonstriert werden.

*Beispiel:*

Es wurde ein Fluidextrakt hergestellt, indem gemahlene Olivenkerne mit Isopropanol extrahiert wurden. 12 kg des nach Entfernung überschüssigen Isopropanols erhaltenen Fluid-Extraktes (1:1), 200 g Menthol, 200 g Pantothenylalkohol und 800 g Parfüm wurden dann mit 50 vol.-%igem Isopropanol auf 100 l aufgefüllt. Das so hergestellte Haarwasser wurde dann an 20 Personen (15 Frauen und 5 Männer unterschiedlichen Alters) getestet.

Es wurde festgestellt, dass das beschriebene Haarwasser als sehr gut verträglich bezeichnet werden konnte. Es stellte sich heraus, dass das Haarwasser im 24-h-Wert in einem Fall eine + + positive, in vier Fällen eine + positive Reaktion, in vier weiteren Fällen eine schwach positive Reaktion und in den restlichen 10 Fällen keine Reaktion zeigte. Im 48-h-Test waren die Reaktionen naturgemäss rückläufig, so dass hier nur in einem Fall zweifach, einem Fall einfach und in zwei Fällen schwach positive Reaktionen beobachtet wurden. Der Rest von 15 Patienten war im 48-h-Wert komplett negativ.

Dieses Ergebnis ist für ein Haarwasser als hervorragend zu bezeichnen, da eine gewisse Reizreaktion erwünscht ist. Somit eignet sich dieses erfindungsgemässe Haarwasser, gemessen an den zahlreichen auf dem Markt befindlichen Kosmetika, gut als Haarpflegemittel.

Ferner wurde festgestellt, dass sich bei mehreren Testpersonen nicht nur das Kopfhaar kräftigte und ein vorhandener Haarausfall gestoppt wurde, sondern auch neuer Haarflaum im Sinne eines neuen Haarwachstums sichtbar wurde. Die aufgrund dieser Beobachtungen vorgenommenen weiteren Prüfungen bestätigten, dass das erfindungsgemässe Haarwasser eine ausgeprägte wachstumsfördernde Wirkung besitzt.

## Patentanspruch

Haarpflegemittel auf Basis üblicher Bestandteile, ausgenommen eine Zusammensetzung, die überwiegend aus Olivenkernöl und im übrigen aus Pfirsichkernöl, Mandelöl und Wegerichöl sowie gegebenenfalls Parfüm wie Fliederöl besteht, dadurch gekennzeichnet, dass es einen unter Verwendung organischer Lösungsmittel hergestellten Extrakt von Oliverkernen, Granatapfelsamen, *Glossostemon bruguieri* und/oder Knollen der *Pueraria mirifica* enthält.

## Claim

Product for the care of hair based on conventional constituents except a composition, the major portion of which consists of oil of olive kernels and the remaining ingredients of which being oil of peach kernels, oil of almonds and oil of plantain as well as optionally perfume like oil of lilac, characterized in that it contains an extract of olive kernels, pomegranate pips, *Glossostemon bruguieri* and/or tubers of *Pueraria mirifica* obtained by using organic solvents.

## Revendication

Produit pour les soins des cheveux à base de constituants habituels, à l'exception d'une composition qui consiste pour le principal en huile de noyaux d'olives et pour le reste en huile de noyaux de pêches, huile d'amandes et huile de plaintain, ainsi qu'éventuellement en parfum tel que de l'essence de lilas, caractérisé en ce qu'il contient un extrait de noyaux d'olives, de graines de grenades, de *Glossostemon bruguieri* et/ou de tubercules de *Pueraria mirifica* préparé au moyen de solvants organiques.